# EUROPÄISCHE PATENTSCHRIFT

(11) **EP 2 625 156 B1**
(45) Veröffentlichungstag und Bekanntmachung des Hinweises auf die Patenterteilung: **21.05.2014**
(21) Anmeldenummer: 11773218.0
(22) Anmeldetag: 05.10.2011
(51) Int. Cl.: C07C 1/06, B01J 19/24

(54) **VERFAHREN UND VORRICHTUNG ZUR ERZEUGUNG EINES METHANREICHEN GASES AUS SYNTHESEGAS**
PROCESS AND APPARATUS FOR PRODUCING A METHANE-RICH GAS FROM SYNTHESIS GAS
PROCÉDÉ ET DISPOSITIF DE PRODUCTION DE GAZ RICHE EN MÉTHANE À PARTIR DE GAZ DE SYNTHÈSE

(30) Priorität: 05.10.2010 DE 102010037980
(43) Veröffentlichungstag der Anmeldung: 14.08.2013
(73) Patentinhaber: ThyssenKrupp Uhde GmbH, 44141 Dortmund (DE)
(72) Erfinder: MENZEL, Johannes, 45731 Waltrop (DE); THIELERT, Holger, 44379 Dortmund (DE)
(74) Vertreter: Albrecht, Rainer Harald
(86) Internationale Anmeldenummer: PCT/EP2011/067369
(87) Internationale Veröffentlichungsnummer: WO 2012/045766

(56) Entgegenhaltungen:
- DE-A1- 2 914 806
- US-A1- 2010 162 627

## Beschreibung

Die Erfindung betrifft ein Verfahren zur Erzeugung eines methanreichen Gases aus Synthesegas, wobei ein CO und H₂ enthaltendes Synthesegas mit einem aus dem methanreichen Produktgas abgezweigten und zurückgeführten Gasstrom gemischt und danach durch ein Katalysatorbett aus einem Methanisierungskatalysator geführt wird, wobei in dem Katalysatorbett eine Methanisierung erfolgt und der Gasstrom durch frei werdende Reaktionswärme aufgeheizt wird. Die Erfindung betrifft ferner einen Methanisierungsreaktor zur Durchführung des Verfahrens.

Als Methanisierungskatalysator werden nickelhaltige Katalysatoren verwendet, deren nickelhaltige Wirksubstanz an Trägermaterialien z. B. aus Aluminiumoxid, Siliciumdioxid, Zirkoniumoxid und dergleichen gebunden sind. Im Katalysatorbett laufen im Wesentlichen die nachfolgenden exothermen Methanisierungsreaktionen ab:

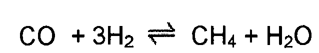

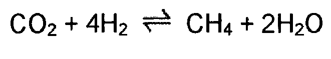

Die Bildung von Methan wird von einer starken Wärmeentwicklung begleitet, so dass die Temperaturen der Reaktanten und der Produkte während des Durchganges durch das Katalysatorbett ansteigen. Dabei nimmt bei steigenden Temperaturen die Gleichgewichtskonzentration von Methan ab. Je höher die Temperatur des das Katalysatorbett verlassenden Gasstromes ist, desto kleiner ist der Methangehalt des Gasstromes entsprechend des nach dem vorstehend angegebenen Reaktionsgleichungen zugrunde liegenden Reaktionsgleichgewichtes. Aus diesem Grunde sollte bei den Methanisierungsreaktionen die Reaktionstemperatur möglichst niedrig gehalten werden. Die Festlegung einer geeigneten Eintrittstemperatur für das Synthesegas in das Katalysatorbett wird jedoch von anderen Kriterien bestimmt. Zu berücksichtigen ist, dass die Reaktionsgeschwindigkeit mit abnehmender Temperatur kleiner wird. Vor allem muss aber auch berücksichtigt werden, dass eine Eintrittstemperatur von weniger als 290 °C zu einer irreversiblen Schädigung des Nickelkatalysators führen kann, was nach gegenwärtigem Kenntnisstand durch eine Reaktion von Nickel mit Kohlenmonoxid zu Nickelcarbonyl hervorgerufen wird.

Bei einem aus DE 29 14 806 A1 bekannten Verfahren, von dem die Erfindung ausgeht, wird dem aus einem Methanisierungskatalysator bestehenden Katalysatorbett eine CO-Konvertierungsstufe vorgeschaltet, um den CO-Gehalt des Feedgases zu reduzieren und den CO-Partialdruck so weit abzusenken, dass es zu keiner den Katalysator schädigenden Carbonylbildung kommen kann. In der CO-Konvertierungsstufe läuft eine katalytische Shift-Reaktion nach folgender Reaktionsgleichung 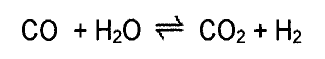 ab. Der Katalysator für die CO-Konvertierungsstufe, auch Shift-Katalysator genannt, enthält beispielsweise zwei der Metalle Cu, Zn und Cr, die ebenfalls auf einem Träger gebunden sind. Bei dem bekannten Verfahren werden der gesamte Synthesegasstrom sowie der zu Kühlzwecken aus dem Produktgas abgezweigte Rückführgasstrom durch die CO-Konvertierungsstufe geführt. Der Shift-Katalysator beansprucht dabei bis zu 75% des gesamten Katalysatorvolumens, der für die Methanisierungsreaktionen und für die CO-Konvertierung insgesamt benötigt wird und erfordert entsprechend einen erheblichen apparatetechnischen Aufwand. Da auch die Shift-Reaktion exotherm abläuft, wird das Synthesegas im Zuge der CO-Konvertierung erwärmt, wodurch die Eintrittstemperatur des Synthesegasstromes in den Methanisierungsreaktor ansteigt, was ebenfalls zu einer steigenden Austrittstemperatur führt, womit eine niedrige Eintrittstemperatur in das katalytische CO-Shift Bett nur zum Teil genutzt werden kann.

Der Erfindung liegt die Aufgabe zugrunde, den Wirkungsgrad des Methanisierungsprozesses zu verbessern.

Die Aufgabe wird erfindungsgemäß durch ein Verfahren nach Anspruch 1 gelöst. Die nachgeordneten Ansprüche 2 bis 12 betreffen vorteilhafte Ausgestaltungen des Verfahrens.

Erfindungsgemäß wird das Katalysatorbett auf mehrere nacheinander durchströmte Methanisierungsstufen aufgeteilt. Das Synthesegas wird entsprechend in Teilströme aufgeteilt, die jeweils dem Katalysatorbett einer zugeordneten Methanisierungsstufe zugeführt werden, wobei das eine Methanisierungsstufe verlassende und durch Methanisierungsreaktionen in dieser Stufe aufgeheizte Gas mit dem kälteren Synthesegasteilstrom für die nachfolgende Methanisierungsstufe gemischt und dabei gekühlt wird und wobei der resultierende Mischgasstrom dem Katalysatorbett der nachfolgenden Methanisierungsstufe als Feedgas zugeführt wird. Im Rahmen der Erfindung liegt es insbesondere auch, die Synthesegasströme unterschiedlich auf die einzelnen Katalysatorstufen aufzuteilen. Besonders vorteilhaft ist es hierbei, die zugeführte Synthesegasmenge von Katalysatorstufe zu Katalysatorstufe sukzessive zu erhöhen, da der Produktgasstrom von der jeweils davorliegenden Methanisierungsstufe auch eine moderierende Funktion, ähnlich wie bei dem zurückgeführten Endgasproduktstrom, auf die Reaktionstemperatur hat.

Durch die mengengestufte Prozessführung ist es möglich, das jeweils aus einer Methanisierungsstufe austretende methanreiche Produktgas mit dem für die nächste Methanisierungsstufe vorgesehenen Synthesegasteilstrom wirksam zu kühlen. Die Synthesegasteilströme werden daher vorzugsweise mit einer Temperatur von weniger als 250 °C, z.B. mit einer Temperatur von etwa 100 °C bis 200 °C, zugeführt und mit dem aus der vorhergehenden Methanisierungsstufe austretenden methanreichen Gas gemischt. Durch diese direkte Kühlung wird ein Großteil der Reaktionswärme der Methanisierungsreaktionen dem Gas entzogen, wodurch die aus dem Produktgas abgezweigte und zurückgeführte Gasmenge verringert werden kann. Ferner kann die Temperaturführung in den Methanisierungsstufen verbessert und die Austrittstemperatur aus den Methanisierungsstufen reduziert werden, was zu einem besseren Gleichgewichtsumsatz der Methanisierungsreaktionen führt. Durch die erfindungsgemäße Aufteilung des Synthesegasstromes auf eine Mehrzahl von nacheinander durchströmten Methanisierungsstufen und Ausnutzung der direkten Gaskühlung können die Methanisierungsreaktionen auf einem niedrigeren Temperaturniveau betrieben werden. Mit zunehmender Anzahl der Methanisierungsstufen sind daher höhere Methanumsätze möglich.

Der aus dem methanreichen Produktgas abgezweigte und zurückgeführte Gasstrom kann dem Katalysatorbett der ersten Methanisierungsstufe zugeführt werden. Eine vorteilhafte Ausgestaltung des erfindungsgemäßen Verfahrens, welches zu einer weiteren Prozessverbesserung beiträgt, sieht vor, dass der aus dem Produktgas abgezweigte und zurückgeführte Gasstrom in Teilströme aufgeteilt wird, die den jeweiligen Methanisierungsstufen zugeführt werden. Ab der zweiten Methanisierungsstufe sollte die Temperatur des zugeführten Feedgas so eingestellt sein, dass sich eine Mischgastemperatur von mindestens 300 °C ergibt. Der zurückgeführte Gasstrom wird zweckmäßig gekühlt. Ferner sind die Temperaturen und Mengenströme der vor einer Methanisierungsstufe zusammengeführten Gasströme unter Berücksichtigung der in der nachfolgenden Methanisierungsstufe ablaufenden Methanisierungsreaktionen so aufeinander abzustimmen, dass das die Methanisierungsstufe verlassende methanreiche Gas eine Austrittstemperatur von 600 °C bis 850 °C, vorzugsweise weniger als 800 °C, aufweist.

Die Menge des der ersten Methanisierungsstufe zugeführten Gasstroms entspricht zweckmäßig dem 2- bis 4-fachen der Menge an Synthesegas, welches als Teilstrom des Gesamtstroms an Synthesegas der ersten Methanisierungsstufe zugeführt wird. Der ersten Methanisierungsstufe kann weniger als 10 %, vorzugsweise 2 % bis 5 % des Synthesegases als Feedgas zugeführt werden.

Eine besonders bevorzugte Ausführung des erfindungsgemäßen Verfahrens sieht vor, dass der ersten Methanisierungsstufe eine CO-Konvertierungsstufe mit einem CO-Konvertierungskatalysator vorgeschaltet wird und dass aus dem Synthesegas ein Synthesegasteilstrom abgezweigt wird, welcher der CO-Konvertierungsstufe zugeführt wird. In der Konvertierungsstufe wird ein Shift-Katalysator verwendet, der jedoch keine Teile enthält, die Metallcarbonyle bilden. Übliche und geeignete Shift-Katalysatoren enthalten Kombinationen der Metalle Kupfer, Zink und Chrom. Erfindungsgemäß wird nicht der gesamte Synthesegasstrom durch die CO-Konvertierungsstufe zugeführt, sondern in vorteilhafter Weise lediglich ein aus dem Synthesegas abgezweigter Teilstrom von weniger als 10 % der gesamten Synthesegasmenge. Vorzugsweise wird nur 2 % bis 5 % des Synthesegases mit einer Eintrittstemperatur von 200 °C bis 250 °C in das aus einem Shift-Katalysator bestehende Katalysatorbett der CO-Konvertierungsstufe eingeleitet. Bei Bedarf wird durch Zugabe von Direktdampf das im Feedgas vorhandene CO zu CO₂ und Wasserstoff umgesetzt. Dadurch wird das in der kleinen Feedgasmenge befindliche CO stark in der Konzentration abgesenkt, so dass eine mögliche Bildung von Nickelcarbonyl in der nachfolgenden Methanisierungsstufe praktisch ausgeschlossen ist.

Neben Prozessdampf kann der CO-Konvertierungsstufe auch ein methanreiches Rückführgas mit vorzugsweise größer 60 vol% zugeführt werden, welches als Produktgas zurückgeführt wird. Dabei kann das Produktgas auch durch weitere, den hier beschriebenen Methanisierungsstufen nachgelagerten Methanisierungsreaktoren entstammen. Der methanreiche Rückführgasstrom, bzw. ein Teilstrom davon kann auch dem die CO-Konvertierungsstufe verlassenden Gasstrom zugemischt werden, wobei gemäß einer bevorzugten Ausgestaltung die Gesamtmenge des zu der ersten CO-Konvertierungsstufe und der ersten Methanisierungsstufe zurückgeführten Gasstroms dem 2- bis 4-fachen der Menge an Synthesegas entspricht, welches als Teilstrom des Gesamtstroms an Synthesegas der CO-Konvertierungsstufe sowie der ersten Methanisierungsstufe zugeführt wird. Das Verhältnis wird im Folgenden auch als Rückflussverhältnis bezeichnet. Durch das erfindungsgemäße große Rückflussverhältnis von vorzugsweise 2 bis 4 wird durch Verdünnung der CO-Gehalt im Mischgas weiter herabgesetzt. Durch eine entsprechende Einstellung des Rücklaufverhältnisses lässt sich eine Mischtemperatur für das Mischgas aus dem Produktgas der CO-Konvertierung, dem zugeführten Synthesegasstrom und dem zurückgeführten Produktgasstrom einstellen, die größer oder gleich der Anspringtemperatur für die nachfolgende Methanisierung ist, und beispielsweise zwischen 250°C und 400°C, je nach eingesetztem Katalysatortyp beträgt.

Gegenstand der Erfindung ist auch ein Methanisierungsreaktor nach Anspruch 16 zur Durchführung des beschriebenen Verfahrens. Die Ansprüche 17 bis 21 beschreiben vorteilhafte Ausgestaltungen des Methanisierungsreaktors.

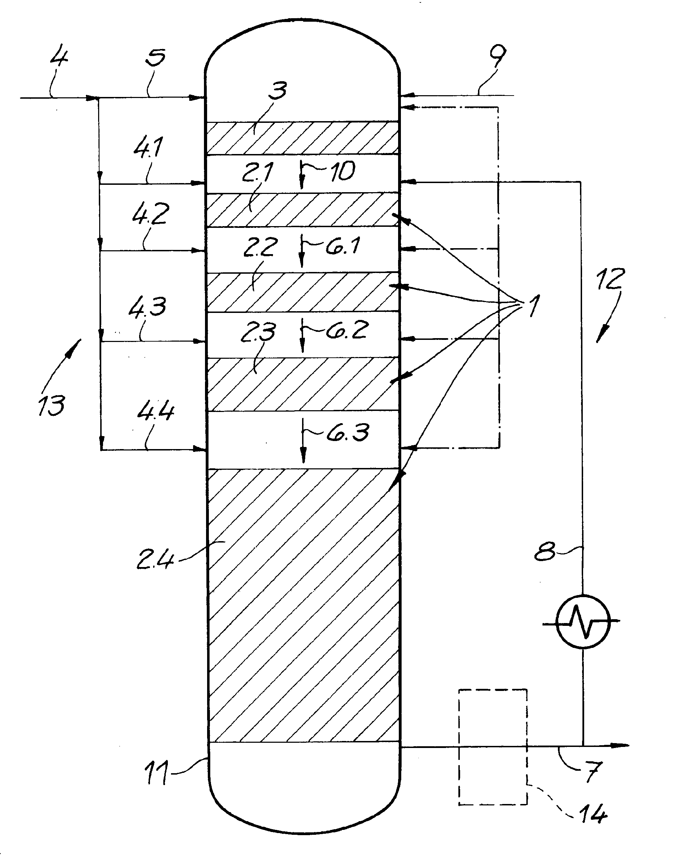

Im Folgenden wird die Erfindung anhand einer lediglich ein Ausführungsbeispiel darstellenden Zeichnung erläutert. Die einzige Figur zeigt schematisch einen Methanisierungsreaktor zur Erzeugung eines methanreichen Gases aus Synthesegas.

Der Methanisierungsreaktor enthält ein Katalysatorbett 1 aus einem nickelhaltigen Methanisierungskatalysator, wobei das Katalysatorbett 1 auf mehrere nacheinander durchströmte Methanisierungsstufen 2.1 bis 2.4 aufgeteilt ist. Der ersten Methanisierungsstufe 2.1 ist eine CO-Konvertierungsstufe 3 mit einem CO-Konvertierungskatalysator, auch Shift-Katalysator genannt, vorgeschaltet. Der Figur entnimmt man, dass ein CO und H₂ enthaltendes Synthesegas 4 in Teilströme 4.1 bis 4.5, 5 aufgeteilt wird, wobei ein Teilstrom 5 der CO-Konvertierungsstufe zugeführt wird und die übrigen Teilströme 4.1 bis 4.4 jeweils dem Katalysatorbett einer zugeordneten Methanisierungsstufe 2.1 bis 2.4 zugeführt werden. Das eine Methanisierungsstufe verlassende und durch Methanisierungsreaktionen in dieser Stufe aufgeheizte Gas 6.1 bis 6.3 wird mit dem Synthesegasteilstrom 4.2 bis 4.4 für die nachfolgende Methanisierungsstufe gemischt und dabei gekühlt. Der resultierende Mischgasstrom wird dem Katalysatorbett der nachfolgenden Methanisierungsstufe als Feedgas zugeführt.

Aus dem methanreichen Produktgas 7 wird ein Gasstrom 8 abgezweigt und in den Methanisierungsreaktor zurückgeführt. Der zurückgeführte Gasstrom 8 wird im Ausführungsbeispiel dem Katalysatorbett der ersten Methanisierungsstufe 2.1 zugeführt. Optional besteht auch die Möglichkeit, dass der aus dem Produktgas 7 abgezweigte und zurückgeführte Gasstrom in Teilströme aufgeteilt wird, die den Methanisierungsstufen 2.1 bis 2.4 zugeordnet sind und diesen zugeführt werden. Der zurückgeführte Gasstrom 8 wird gekühlt.

Die Mengenströme und Temperaturen der Gasströme werden so aufeinander abgestimmt, dass die Mischgasströme am Eintritt der zweiten und jeder weiteren Methanisierungsstufe 2.2 bis 2.4 eine Mischgastemperatur von mindestens 300 °C aufweisen. Ferner werden die Temperaturen und Mengenströme der vor einer Methanisierungsstufe zusammengeführten Gasströme unter Berücksichtigung der in der nachfolgenden Methanisierungsstufe ablaufenden Methanisierungsreaktionen so aufeinander abgestimmt, dass das die Methanisierungsstufe verlassende methanreiche Gas 6.1 bis 6.3 eine Austrittstemperatur von 600 °C bis 850 °C, vorzugsweise etwa 800 °C, aufweist.

Die Menge des der CO-Konvertierungsstufe zugeführten Synthesegasteilstromes 5 liegt vorzugsweise im Bereich zwischen 2% und 5% des insgesamt zugeführten Synthesegases 4 als Feedgas. Durch Zugabe von Wasserdampf (Direktdampf) 9 wird das im Feedgas vorhandene CO zu CO₂ und Wasserstoff umgesetzt. Der CO-Gehalt des Gasstromes 10 wird durch die CO-Konvertierung so weit reduziert, dass es in der nachfolgenden Methanisierungsstufe 2.1 zu keiner den Katalysator schädigenden Nickelcarbonylbildung kommen kann.

Der die CO-Konvertierungsstufe 3 verlassende Gasstrom 10 wird mit einem aus dem methanreichen Produktgas abgezweigten Rückführgasstrom 8 gemischt, wobei der Rückführgasstrom 8 das Zwei- bis Vierfache der Menge des Gasstromes beträgt, der sich aus der Mischung des die CO-Konvertierungsstufe verlassenden Gasstromes 10 und des der ersten Methanisierungsstufe 2.1 zugeführten Synthesegasteilstromes 4.1 ergibt. Das Verhältnis wird im Folgenden auch als Rückflussverhältnis bezeichnet. Durch das gewählte Rückflussverhältnis kann der CO-Gehalt in dem Mischgas, welches der ersten Methanisierungsstufe 2.1 zugeführt wird, so weit abgesenkt werden, dass eine Schädigung des Methanisierungskatalysators durch Nickelcarbonylbildung ausgeschlossen ist.

Das Mischgas wird nun dem unterhalb des CO-Konvertierungskatalysators 3 angeordneten Katalysatorbett der ersten Methanisierungsstufe 2.1 zugeführt. Hier kommt es zu einer Methanisierung, die mit einer Aufheizung des Gasstromes auf 600 °C bis 800°C verbunden ist. Dem methanreichen Produktgas, welches das Katalysatorbett der ersten Methanisierungsstufe 2.1 verlässt, wird ein weiterer Synthesegasteilstrom 4.2 zugemischt, wobei eine Mischtemperatur eingestellt wird, die mehr als 300 °C beträgt. Dadurch wird sichergestellt, dass es zu keiner Schädigung des Methanisierungskatalysators durch Nickelcarbonylbildung kommt. Zur weiteren Einstellung der Mischgastemperatur oder auch zur Absenkung der Austrittstemperatur aus der nachfolgenden Methanisierungsstufe 2.2 kann dem Mischgas auch ein aus dem Rückführgas 8 abgezweigter Teilstrom zugeführt werden, bevor das Mischgas der zweiten Methanisierungsstufe 2.2 zugeführt wird. Auch die zweite Methanisierungsstufe 2.2 weist ein Katalysatorbett aus einem Methanisierungskatalysator auf. Dabei kommt es zu einer Methanisierung und einem Temperaturanstieg im Gasstrom.

Das beschriebene Verfahren wiederholt sich in einer oder mehreren weiteren Methanisierungsstufen.

Der in der Figur dargestellte Methanisierungsreaktor zur Durchführung des beschriebenen Verfahrens umfasst ein Gehäuse 11, mehrere innerhalb des Gehäuses angeordnete Methanisierungsstufen 2.1 bis 2.4, die jeweils ein Katalysatorbett aus einem Methanisierungskatalysator aufweisen, mehrere Synthesegaseinlässe für die Zuführung von Synthesegas 4, einen Produktgasauslass sowie eine Einrichtung 12 zur Gasrückführung eines Produktgasteilstromes zur Einlassseite der ersten Methanisierungsstufe 2.1. In Durchströmungsrichtung ist jeweils vor jeder Methanisierungsstufe 2.1 bis 2.4 mindestens ein Synthesegaseinlass angeordnet. Die Synthesegaseinlässe sind durch einen Strömungsverteiler 13 verbunden, welcher das Synthesegas 4 in Synthesegasteilströme 4.1 bis 4.4 aufteilt und den Methanisierungsstufen 2.1 bis 2.4 zuführt.

Der Darstellung entnimmt man, dass das Gehäuse 11 einen stehenden und von oben nach unten durchströmten Apparat bildet, wobei zwischen den Methanisierungsstufen 2.1 bis 2.4 katalysatorfreie Räume angeordnet sind, an welche die Synthesegaseinlässe angeschlossen sind. Die katalysatorfreien Räume können Mischungselemente, wie z. B. Kugelschüttungen, Füllkörperschüttungen und dergleichen aus Inertmaterial enthalten, die dafür sorgen, dass die jeweiligen Produktgase aus den einzelnen Katalysatorschüttungen sich mit den zugeführten Gasen gut vermischen.

An die Einrichtung 12 zur Gasrückführung können Abzweigleitungen anschließen, wobei in Durchströmungsrichtung vor den Methanisierungsstufen 2.1 bis 2.4 jeweils zumindest eine Abzweigleitung in das Gehäuse 11 mündet.

In Durchströmungsrichtung vor und im Ausführungsbeispiel oberhalb der ersten Methanisierungsstufe 2.1 ist eine CO-Konvertierungsstufe 3 angeschlossen, die eine Katalysatorschicht aus einem CO-Konvertierungskatalysator aufweist. In Strömungsrichtung vor dem Katalysatorbett der Konvertierungsstufe 3 ist ein Synthesegaseinlass angeordnet, durch den ein Synthesegasteilstrom 5 zuführbar ist. Ferner mündet in den Bereich vor dem Katalysatorbett des CO-Konvertierungskatalysators eine Dampfleitung für die Zuführung von Direktdampf 9 an. In dem Ausführungsbeispiel schließt an die Einrichtung 12 auch eine Abzweigleitung an, welche in Durchströmrichtung vor der Konvertierungsstufe 3 in das Gehäuse mündet und es ermöglicht auch die Konvertierungsstufe mit einem Anteil des Rückführgases zu beaufschlagen. Die Vermischung der zugeführten Gase (5, 4.1 bis 4.4, 12) mit den aus den jeweiligen Katalysatorbetten austretenden Gasströmen (10, 6.1 bis 6.3) erfolgt über Mischungselemente wie z. B. einer Schüttung aus Inertkugeln oder anderen Mischelementen. Aufgrund des von oben nach unten zunehmenden Gasmengenstromes nimmt die für den Synthesegasumsatz benötigte Katalysatorbetthöhe immer weiter zu, wobei die ersetzten Betten aufgrund der anfangs kleinen Gasmenge auch entsprechend wenig Katalysator benötigen. Dies gilt insbesondere auch für das CO-Shift Katalysatorbett.

Das Rückführgas 8 wird in Strömungsrichtung gesehen hinter den zuvor beschriebenen Methanisierungsstufen 2.1 bis 2.4, also hinter dem Gehäuse 11 abgeführt. In der einzigen Figur ist in diesem Zusammenhang angedeutet, dass das Rückführgas 8 vor seiner Ausschleusung auch durch weitere nur schematische Methanisierungsreaktoren 14 geführt werden kann. Des Weiteren versteht es sich, dass die Leitungssysteme mit Zuführleitungen, Abzweigleitungen und dergleichen nur rein schematisch dargestellt sind und insbesondere Mittel zur Durchflusssteuerung aufweisen können, um die gewünschten Strömungsverhältnisse einstellen zu können.

## Patentansprüche

1. Verfahren zur Erzeugung eines methanreichen Gases aus Synthesegas, wobei ein CO und H₂ enthaltende Synthesegas mit einem aus dem methanreichen Produktgas (7) abgezweigten und zurückgeführten Gasstrom (8) gemischt und danach durch ein Katalysatorbett (1) aus einem Methanisierungskatalysator geführt wird, wobei in dem Katalysatorbett (1) eine Methanisierung erfolgt und der Gasstrom durch frei werdende Reaktionswärme aufgeheizt wird, **dadurch gekennzeichnet, dass** das Katalysatorbett (1) auf mehrere nacheinander durchströmte Methanisierungsstufen (2.1 bis 2.4) aufgeteilt wird und dass das Synthesegas (4) entsprechend in Teilströme (4.1 bis 4.4) aufgeteilt wird, die jeweils dem Katalysatorbett einer zugeordneten Methanisierungsstufe (2.1 bis 2.4) zugeführt werden, wobei das eine Methanisierungsstufe verlassende und durch Methanisierungsreaktionen in dieser Stufe aufgeheizte Gas (6.1 bis 6.3) mit dem Synthesegasteilstrom (4.2 bis 4.4) für die nachfolgende Methanisierungsstufe gemischt und dabei gekühlt wird und wobei der resultierende Mischgasstrom dem Katalysatorbett (1) der nachfolgenden Methanisierungsstufe als Feedgas zugeführt wird.

2. Verfahren nach Anspruch 1, **dadurch gekennzeichnet, dass** der aus dem methanreichen Produktgas (7) abgezweigte und zurückgeführte Gasstrom (8) zumindest dem Katalysatorbett der ersten Methanisierungsstufe (2.1) zugeführt wird.

3. Verfahren nach Anspruch 2, **dadurch gekennzeichnet, dass** der aus dem methanreichen Produktgas (7) abgezweigte und zurückgeführte Gasstrom (8) in Teilströme aufgeteilt wird, die den Methanisierungsstufen (2.1 bis 2.4) zugeführt werden.

4. Verfahren nach einem der Ansprüche 1 bis 3, **dadurch gekennzeichnet, dass** in dem einer Methanisierungsstufe (2.1 bis 2.4) zugeführten Feedgas eine Mischgastemperatur von mindestens 300 °C eingestellt wird.

5. Verfahren nach einem der Ansprüche 1 bis 4, **dadurch gekennzeichnet, dass** die Menge des zu der ersten Methanisierungsstufe (2.1) zugeführten Gasstromes (8) dem 2- bis 4-fachen der Menge an Synthesegas (4) entspricht, welches als Teilstrom (4.1) des Gesamtstroms an Synthesegas (4) der ersten Methanisierungsstufe (2.1) zugeführt wird.

6. Verfahren nach einem der Ansprüche 1 bis 5, **dadurch gekennzeichnet, dass** der ersten Methanisierungsstufe (2.1) weniger als 10 %, vorzugsweise 2 % bis 5 % des Synthesegases (4), als Feedgas zugeführt wird.

7. Verfahren nach einem der Ansprüche 1 bis 5, **dadurch gekennzeichnet, dass** die Temperaturen und Mengenströme der vor einer Methanisierungsstufe (2.1 bis 2.4) zusammengeführten Gasströme unter Berücksichtigung der in der nachfolgenden Methanisierungsstufe ablaufenden Methanisierungsreaktionen so aufeinander abgestimmt werden, dass das die Methanisierungsstufe verlassende methanreiche Gas eine Austrittstemperatur von 600 °C bis 850 °C, vorzugsweise weniger als 800 °C, aufweist.

8. Verfahren nach einem der Ansprüche 1 bis 7, **dadurch gekennzeichnet, dass** der ersten Methanisierungsstufe (2.1) eine CO-Konvertierungsstufe (3) mit einem CO-Konvertierungskatalysator vorgeschaltet wird und dass aus dem Synthesegas (4) ein Synthesegasteilstrom (5) abgezweigt wird, welcher der CO-Konvertierungsstufe (3) zugeführt wird.

9. Verfahren nach Anspruch 8, **dadurch gekennzeichnet, dass** der CO-Konvertierungsstufe (3) Wasserdampf (9) zugeführt wird.

10. Verfahren nach Anspruch 8 oder 9, **dadurch gekennzeichnet, dass** der CO-Konvertierungsstufe (3) ein Teilstrom des zurückgeführten Gasstroms zugeführt wird

11. Verfahren nach einem der Ansprüche 8 bis 10, **dadurch gekennzeichnet, dass** die Gesamtmenge des zu der CO-Konvertierungsstufe (3) und der ersten Methanisierungsstufe (2.1) zurückgeführten Gasstroms (8) dem zwei- bis vierfachen der Menge an Synthesegas (4) entspricht, welches als Teilstrom (5, 4.1) des Gesamtstroms an Synthesegas (4) der CO-Konvertierungsstufe (3) sowie der ersten Methanisierungsstufe (2.1) zugeführt wird.

12. Verfahren nach einem der Ansprüche 8 bis 11, **dadurch gekennzeichnet, dass** der Synthesegasteilstrom (5) mit einer Temperatur von 200 °C bis 250 °C der CO-Konvertierungsstufe (3) zugeführt wird.

13. Verfahren nach einem der Ansprüche 8 bis 12, **dadurch gekennzeichnet, dass** der Konvertierungsstufe (3) weniger als 10%, vorzugsweise 2% bis 5%, des Synthesegases (4) als Feedgas zugeführt wird.

14. Verfahren nach einem der Ansprüche 8 bis 13, **dadurch gekennzeichnet, dass** dem die CO-Konvertierungsstufe verlassenden Gasstrom sowie dem die Methanisierungsstufen (2.1 bis 2.3) verlassenden Gas vor Eintritt in die nachfolgende Methanisierungsstufe (2.1 bis 2.4) derart ein Synthesegasteilstrom (4.1 bis 4.4) und optional zumindest ein Anteil des zurückgeführten Gasstroms (8) in solchen Mengen beigemischt wird, dass sich eine Mischtemperatur ergibt, die größer oder gleich der Anspringtemperatur für die Methanisierung in der jeweils nachfolgenden Methanisierungsstufe (2.1 bis 2.4) ist, wobei die sich ergebende Mischgastemperatur bevorzugt größer als 300°C ist.

15. Verfahren nach einem der Ansprüche 1 bis 14, **dadurch gekennzeichnet, dass** die Synthesegasteilströme (4.1 bis 4.4) zur Kühlung des aus den Methanisierungsstufen (2.1 bis 2.4) austretenden methanreichen Gases mit einer Temperatur von weniger als 250 °C zugeführt werden.

16. Methanisierungsreaktor zur Durchführung des Verfahrens nach einem der Ansprüche 1 bis 15, mit
einem Gehäuse (11),
mehreren innerhalb des Gehäuses (11) angeordneten Methanisierungsstufen (2.1 bis 2.4), die jeweils ein Katalysatorbett aus einem Methanisierungskatalysator aufweisen,
mehreren Synthesegaseinlässen für die Zuführung von Synthesegas (4), einem Produktgasauslass,
einer Einrichtung (12) zur Gasrückführung eines Produktgasteilstromes zur Einlassseite der ersten Methanisierungsstufe (2.1),
wobei in Durchströmungsrichtung jeweils vor jeder Methanisierungsstufe (2.1 bis 2.4) mindestens ein Synthesegaseinlass angeordnet ist und wobei die Synthesegaseinlässe durch einen Strömungsverteiler (13) verbunden sind, der das Synthesegas (4) in Synthesegasteilströme (4.1 bis 4.4) aufteilt und den Methanisierungsstufen (2.1 bis 2.4) zuführt.

17. Methanisierungsreaktor nach Anspruch 16, **dadurch gekennzeichnet, dass** das Gehäuse (11) einen stehenden und von oben nach unten durchströmten Apparat bildet und dass zwischen den Methanisierungsstufen (2.1 bis 2.4) katalysatorfreie Räume angeordnet sind, an welche die Synthesegaseinlässe angeschlossen sind.

18. Methanisierungsreaktor nach Anspruch 17, **dadurch gekennzeichnet, dass** die katalysatorfreien Räume Mischungselemente, wie z. B. Kugelschüttungen oder Füllkörperschüttungen aus Inertmaterial enthalten, die dafür sorgen, dass die jeweiligen Produktgase aus den einzelnen Katalysatorschüttungen sich mit den zugeführten Gasen gut vermischen.

19. Methanisierungsreaktor nach einem der Ansprüche 16 bis 18, **dadurch gekennzeichnet, dass** an die Einrichtung (12) zur Gasrückführung Abzweigleitungen anschließen und dass in Durchströmungsrichtung vor den Methanisierungsstufen (2.1 bis 2.4) jeweils zumindest eine Abzweigleitung in das Gehäuse (11) mündet.

20. Methanisierungsreaktor nach einem der Ansprüche 16 bis 19, **dadurch gekennzeichnet, dass** in Durchströmungsrichtung vor der ersten Methanisierungsstufe (2.1) eine CO-Konvertierungsstufe (3) angeschlossen ist, die ein Katalysatorbett aus einem CO-Konvertierungskatalysator aufweist, und dass in Strömungsrichtung vor dem Katalysatorbett der CO-Konvertierungsstufe (3) ein Synthesegaseinlass angeordnet ist, durch den ein Synthesegasteilstrom (5) zuführbar ist.

21. Methanisierungsreaktor nach Anspruch 20, **dadurch gekennzeichnet, dass** an die Einrichtung (12) zur Gasrückführung zumindest eine Abzweigleitung anschließt, die in Durchströmungsrichtung vor der CO-Konvertierungsstufe (3) in das Gehäuse mündet.

## Claims

1. A method for producing a methane-rich gas from synthesis gas, wherein a synthesis gas containing CO and H₂ is mixed with a gas stream (8) diverted from the methane-rich product gas (7) and returned thereto, then passed through a catalyst bed (1) formed by a methanation catalyst so that methanation is carried out in the catalyst bed (1) and the gas stream is heated by the released heat of reaction, **characterized in that** the catalyst bed (1) is divided into a plurality of methanation stages (2.1 to 2.4) through which the gas flows sequentially and **in that** the synthesis gas (4) is correspondingly divided into sub-streams(4.1 to 4.4) which are respectively fed to the catalyst bed of an associated methanation stage (2.1 to 2.4), wherein the gas (6.1 to 6.3) which exits a methanation stage and which has been heated up by methanation reactions in this stage is mixed with the sub-stream of synthesis gas (4.2 to 4.4) for the subsequent methanation stage and cooled thereby, and wherein the resulting mixed gas stream is fed to the catalyst bed (1) of the subsequent methanation stage as feed gas.

2. The method according to claim 1, **characterized in that** the gas stream (8) which has been diverted from and returned to the methane-rich product gas (7) is fed to at least the catalyst bed of the first methanation stage (2.1).

3. The method according to claim 2, **characterized in that** the gas stream (8) which has been diverted from and returned to the methane-rich product gas (7) is divided into sub-streams which are fed to the methanation stages (2.1 to 2.4).

4. The method according to any one of claims 1 to 3, **characterized in that** the feed gas which is fed to a methanation stage (2.1 to 2.4) is set at a mixed gas temperature of at least 300°C.

5. The method according to any one of claims 1 to 4, **characterized in that** the quantity of the gas stream (8) fed to the first methanation stage (2.1) is 2 to 4 times greater than the quantity of synthesis gas (4) which is fed to the first methanation stage (2.1) as a sub-stream (4.1) of the total stream of synthesis gas (4).

6. The method according to any one of claims 1 to 5, **characterized in that** less than 10%, preferably 2% to 5% of the synthesis gas (4) is fed to the first methanation stage (2.1) as feed gas.

7. The method according to any one of claims 1 to 5, **characterized in that** the temperatures and flows of the gas streams which are combined before a methanation stage (2.1 to 2.4) are set with respect to each other in such a manner, taking into account the methanation reactions that take place in the subsequent methanation stage, that the methane-rich gas leaving the methanation stage has an outlet temperature of 600°C to 850°C, preferably less than 800°C.

8. The method according to any one of claims 1 to 7, **characterized in that** a CO conversion stage (3) with a CO conversion catalyst is connected upstream of the first methanation stage (2.1) and **in that** a synthesis gas sub-stream (5) is diverted from the synthesis gas (4) and fed to the CO conversion stage (3).

9. The method according to claim 8, **characterized in that** steam (9) is fed to the CO conversion stage (3).

10. The method according to claim 8 or 9, **characterized in that** a sub-stream of the returned gas stream is fed to the CO conversion stage (3).

11. The method according to any one of claims 8 to 10, **characterized in that** the total quantity of the gas stream (8) returned to the CO conversion stage (3) and the first methanation stage (2.1) is equivalent to 2 to 4 times the quantity of synthesis gas (4) which is fed to the CO conversion stage (3) and the first methanation stage (2.1) as a sub-stream (5, 4.1) of the total synthesis gas stream (4).

12. The method according to any one of claims 8 to 11, **characterized in that** the synthesis gas sub-stream (5) is fed to the CO conversion stage (3) at a temperature of 200°C to 250°C.

13. The method according to any one of claims 8 to 12, **characterized in that** less than 10%, preferably 2% to 5%, of the synthesis gas (4) is fed to the conversion stage (3) as feed gas.

14. The method according to any one of claims 8 to 13, **characterized in that** the gas stream leaving the CO conversion stage and the gas leaving the methanation stages (2.1 to 2.3) are mixed with a synthesis gas sub-stream (4.1 to 4.4) and optionally at least a portion of the returned gas stream (8) before entering the subsequent methanation stage (2.1 to 2.4) in such a manner that a mixture temperature is obtained which is greater than or equal to the to light-off temperature for the methanation in the subsequent methanation stage (2.1 to 2.4), wherein the resulting mixed gas temperature is preferably greater than 300°C.

15. The method according to any one of claims 1 to 14, **characterized in that** the sub-streams (4.1 to 4.4) of synthesis gas are supplied at a temperature of less than 250°C to cool the methane-rich gas leaving the methanation stages (2.1 to 2.4).

16. A methanation reactor for carrying out the method according to any one of claims 1 to 15, having
a housing (11),
a plurality of methanation stages (2.1 to 2.4) arranged inside the housing (11), each having a catalyst bed comprising a methanation catalyst,
a plurality of synthesis gas inlets for supplying synthesis gas (4),
a product gas outlet,
a device (12) for returning a product gas sub-stream to the inlet side of the first methanation stage (2.1),
wherein at least one respective synthesis gas inlet is disposed upstream of each methanation stage (2.1 to 2.4) and wherein the synthesis gas inlets are interconnected via a flow distributor (13) which divides the synthesis gas (4) into synthesis gas sub-streams (4.1 to 4.4) and feeds them to the methanation stages (2.1 to 2.4).

17. The methanation reactor according to claim 16, **characterized in that** the housing (11) forms a vertical apparatus which is traversed from top to bottom, and chambers without catalysts to which the synthesis gas inlets are connected are disposed between the methanation stages (2.1 to 2.4).

18. The methanation reactor according to claim 17, **characterized in that** the chambers without catalysts contain mixing elements such as beds of beads or beds of packing formed from inert material, which ensure that the respective product gases from the individual catalyst beds are mixed well with the gases which are supplied.

19. The methanation reactor according to any one of claims 16 to 18, **characterized in that** branch lines connect the device (12) for returning the gas, and **in that** at least one of the branch lines upstream of each methanation stage (2.1 to 2.4) opens into the housing (11).

20. The methanation reactor according to any one of claims 16 to 19, **characterized in that** a CO conversion stage (3) is connected upstream of the first methanation stage (2.1), which comprises a catalyst bed formed by a CO conversion catalyst, and **in that** a synthesis gas inlet is disposed upstream of the catalyst bed of the CO conversion stage (3) through which a synthesis gas sub-stream (5) can be fed.

21. The methanation reactor according to claim 20, **characterized in that** at least one branch line which opens into the housing upstream of the CO conversion stage (3) is connected to the device (12) for returning the gas.

## Revendications

1. Procédé de production d'un gaz riche en méthane à partir de gaz de synthèse, lors duquel on mélange un gaz de synthèse contenant du CO et du H₂ avec un flux gazeux (8) dérivé du produit gazeux (7) riche en méthane et recyclé et on le conduit ensuite à travers un lit catalytique (1) en un catalyseur de méthanisation, dans le lit catalytique (1) s'effectuant une méthanisation et on réchauffe le flux gazeux par une chaleur réactive qui se libère, **caractérisé en ce qu'**on divise le lit catalytique (1) en plusieurs étapes de méthanisation (2.1 à 2.4) successivement traversées et **en ce qu'**on divise en conséquence le gaz de synthèse (4) en flux partiels (4.1 à 4.4) que l'on amène chacun au lit catalytique d'une étape de méthanisation (2.1 à 2.4) associée, alors qu'on mélange le gaz (6.1 à 6.3) quittant une étape de méthanisation et réchauffé dans ladite étape par des réactions de méthanisation avec le flux partiel (4.2 à 4.4) de gaz de synthèse pour l'étape de méthanisation suivante et qu'on le refroidit à cet effet et alors qu'on amène le flux de gaz mixte qui en résulte en tant que gaz d'alimentation vers le lit catalytique (1) de l'étape de méthanisation suivante.

2. Procédé selon la revendication 1, **caractérisé en ce qu'**on amène le flux gazeux (8) dérivé du produit gazeux (7) riche en méthane et recyclé au moins vers le lit catalytique de la première étape de méthanisation (2.1).

3. Procédé selon la revendication 2, **caractérisé en ce qu'**on divise le flux gazeux (8) dérivé du produit gazeux (7) riche en méthane et recyclé en flux partiels que l'on amène vers les étapes de méthanisation (2.1 à 2.4).

4. Procédé selon l'une quelconque des revendications 1 à 3, **caractérisé en ce que** dans le gaz d'alimentation amené vers une étape de méthanisation (2.1 à 2.4), on règle une température de gaz mixte d'au moins 300°C.

5. Procédé selon l'une quelconque des revendications 1 à 4, **caractérisé en ce que** la quantité du flux gazeux (8) amené vers la première étape de méthanisation (2.1) correspond à de 2 à 4 fois la quantité de gaz de synthèse (4) qu'on amène en tant que flux partiel (4.1) du flux total de gaz de synthèse (4) à la première étape de méthanisation (2.1).

6. Procédé selon l'une quelconque des revendications 1 à 5, **caractérisé en ce qu'**on amène vers la première étape de méthanisation (2.1) moins de 10 %, de préférence de 2 % à 5 % du gaz de synthèse (4) en tant que gaz d'alimentation.

7. Procédé selon l'une quelconque des revendications 1 à 5, **caractérisé en ce qu'**on adapte les uns aux autres les températures et flux quantitatifs des flux gazeux réunis avant une étape de méthanisation (2.1 à 2.4), sous considération des réactions de méthanisation se déroulant dans l'étape de méthanisation suivante, de telle sorte que le gaz riche en méthane quittant l'étape de méthanisation présente une température de sortie de 600°C à 850°C, de préférence inférieure à 800°C.

8. Procédé selon l'une quelconque des revendications 1 à 7, **caractérisé en ce qu'**on monte en amont de la première étape de méthanisation (2.1) une étape de conversion de CO (3) avec un catalyseur convertisseur de CO et **en ce qu'**on dérive du gaz de synthèse (4) un flux partiel de gaz de synthèse (5) que l'on amène vers l'étape de conversion de CO (3).

9. Procédé selon la revendication 8, **caractérisé en ce qu'**on amène de la vapeur d'eau (9) vers l'étape de conversion de CO (3).

10. Procédé selon la revendication 8 ou 9, **caractérisé en ce qu'**on amène vers l'étape de conversion de CO (3) un flux partiel du flux gazeux recyclé.

11. Procédé selon l'une quelconque des revendications 8 à 10, **caractérisé en ce que** la quantité totale du flux gazeux (8) recyclé vers l'étape de conversion de CO (3) et vers la première étape de méthanisation (2.1) correspond à de 2 fois à 4 fois la quantité de gaz de synthèse (4) que l'on amène en tant que flux partiel (5, 4.1) du flux total de gaz de synthèse (4) vers l'étape de conversion de CO (3), ainsi que vers la première étape de méthanisation (2.1).

12. Procédé selon l'une quelconque des revendications 8 à 11, **caractérisé en ce qu'**on amène le flux partiel (5) de gaz de synthèse à une température de 200°C à 250°C vers l'étape de conversion de CO (3).

13. Procédé selon l'une quelconque des revendications 8 à 12, **caractérisé en ce qu'**on amène vers la couche de conversion (3) moins de 10 %, de préférence de 2 % à 5 % du gaz de synthèse (4) en tant que gaz d'alimentation.

14. Procédé selon l'une quelconque des revendications 8 à 13, **caractérisé en ce qu'**avant l'entrée dans l'étape de méthanisation (2.1 à 2.4) suivante, on mélange au flux gazeux quittant l'étape de conversion de CO, ainsi qu'au gaz quittant les étapes de méthanisation (2.1 à 2.3) un flux partiel de gaz de synthèse (4.1 à 4.4) de façon telle et en option au moins une part du flux gazeux (8) recyclé dans des quantités telles qu'il en résulte une température de mélange qui est supérieure ou égale à la température de démarrage pour la méthanisation dans l'étape de méthanisation (2.1 à 2.4) chaque fois suivante, la température du gaz mixte qui en résulte étant de préférence supérieure à 300°C.

15. Procédé selon l'une quelconque des revendications 1 à 14, **caractérisé en ce que** pour refroidir le gaz riche en méthane sortant des étapes de méthanisation (2.1 à 2.4), on amène les flux partiels (4.1 à 4.4) de gaz de synthèse à une température inférieure à 250°C.

16. Réacteur de méthanisation pour réaliser le procédé selon l'une quelconque des revendications 1 à 15, avec
un corps (11),
plusieurs étapes de méthanisation (2.1 à 2.4) placées à l'intérieur du corps (11), qui comportent chacune un lit catalytique en un catalyseur de méthanisation,
plusieurs entrées de gaz de synthèse pour l'amenage de gaz de synthèse (4),
une sortie de produit gazeux,
un système (12) pour recycler du gaz d'un flux partiel de produit gazeux vers le côté entrée de la première étape de méthanisation (2.1),
dans la direction de circulation, chaque fois avant chaque étape de méthanisation (2.1 à 2.4) étant placée au moins une entrée de gaz de synthèse et les entrées de gaz de synthèse étant reliées par un distributeur de flux (13), qui divise le gaz de synthèse (4) en flux partiels (4.1 à 4.4) de gaz de synthèse et les amène vers les étapes de méthanisation (2.1 à 2.4).

17. Réacteur de méthanisation selon la revendication 16, **caractérisé en ce que** le corps (11) forme un appareil debout et traversé du haut vers le bas et **en ce qu'**entre les étapes de méthanisation (2.1 à 2.4) sont placés des espaces exempts de catalyseur sur lesquels se raccordent les entrées de gaz de synthèse.

18. Réacteur de méthanisation selon la revendication 17, **caractérisé en ce que** les espaces exempts de catalyseur contiennent des éléments mélangeurs, comme par exemple des billes en vrac ou des garnissages de colonne en matière inerte qui assurent que les produits gazeux concernés issus de chacun des débits de catalyseur se mélangent bien avec les gaz amenés.

19. Réacteur de méthanisation selon l'une quelconque des revendications 16 à 18, **caractérisé en ce que** sur le système (12) de recyclage de gaz se raccordent des conduits de dérivation et **en ce qu'**avant les étapes de méthanisation (2.1 à 2.4), dans la direction de circulation, chaque fois au moins un conduit de dérivation débouche dans le corps (11).

20. Réacteur de méthanisation selon l'une quelconque des revendications 16 à 19, **caractérisé en ce qu'**avant l'étape de méthanisation (2.1) dans la direction de circulation est raccordée une étape de conversion de CO (3) qui comporte un lit catalytique en un catalyseur convertisseur de CO et **en ce qu'**avant le lit catalytique de l'étape de conversion de CO (3), dans la direction d'écoulement est placée une entrée de gaz de synthèse à travers laquelle un flux partiel (5) de gaz de synthèse peut être amené.

21. Réacteur de méthanisation selon la revendication 20, **caractérisé en ce qu'**au système (12) de recyclage de gaz se raccorde au moins un conduit de dérivation qui avant l'étape de conversion de CO (3) dans la direction de circulation débouche dans le corps.
